# EUROPEAN PATENT SPECIFICATION

(11) **EP 2 921 197 B1**
(45) Date of publication and mention of the grant of the patent: **08.01.2020**
(21) Application number: 15159468.6
(22) Date of filing: 17.03.2015
(51) Int. Cl.: A61M 25/09

(54) **GUIDEWIRE**
FÜHRUNGSDRAHT
FIL GUIDE

(30) Priority: 19.03.2014 JP 2014055799
(43) Date of publication of application: 23.09.2015
(73) Proprietor: ASAHI INTECC CO., LTD., Seto-shi, Aichi 489-0071 (JP)
(72) Inventor: Nakagawa, Yumiko c/o ASAHI INTECC CO., LTD., Seto-shi, Aichi 489-0071 (JP)
(74) Representative: Winter, Brandl, Fürniss, Hübner, Röss, Kaiser, Polte - Partnerschaft mbB

(56) References cited:
- EP-A2- 0 826 389
- EP-A2- 1 243 283
- DE-A1-102005 022 688
- US-A1- 2002 074 051
- US-A1- 2004 215 109
- US-A1- 2007 010 762

## Description

### BACKGROUND OF THE INVENTION

### 1. Field of the Invention

The present invention relates to a guidewire that is used as a medical device that is inserted into a body cavity for the purpose of treatment or examination.

### 2. Description of the Related Art

Hitherto, various guidewires have been proposed as guidewires for guiding, for example, a catheter that is used by being inserted into body tissues or tubular organs, such as blood vessels, the alimentary canal, or ureters, for performing treatment or examination.

For example, Japanese Unexamined Patent Application Publication No. 2007-90097 (Patent Literature (PTL) 1) discloses a guidewire including a shaft whose diameter gradually increases towards a proximal end side and a coil that is wound around a tip portion of the shaft. In the guidewire, an intermediate portion of the coil is secured to the shaft with a securing material.

US 2007/0010762 A1 describes a steerable guide wire including a core wire, a multi-filament bundle affixed to the distal end of the core, an outer coil surrounding at least a portion of the core wire and the multi-filament bundle and an end cap at the distal end such as a weld, braze, solder, adhesive or the like securing the coil to the multi-filament bundle.

US 2002/0074051 A1 discloses a guidewire comprising a distal end, a shaft portion and a proximal end, wherein the shaft portion comprises at least one helically wound group of at least two wires extending side by side and having a pitch angle in the range of 35° to 76°.

DE 10 2005 022 688 A1 discloses a guide wire for a medical instrument, which has a guide wire core and a sheathing that surrounds the guide wire core at least in sections. According to the invention, the sheathing, at least in a shaft section joined to an end section, is provided with a rigidity greater than that of the guide wire core.

### SUMMARY OF THE INVENTION

Here, for example, when inserting the guidewire that is discussed in PTL 1 (that is, the guidewire including a shaft whose diameter gradually increases towards the proximal end side) along an inverted U-shaped path extending from a blood vessel in the right leg to a blood vessel in the left leg by a cross over method, in addition to the operability of the guidewire being reduced due to a difficulty in following the blood vessels in the legs that are considerably bent, the shaft may be excessively bent due to a load that is received by the shaft when the shaft, for example, contacts vascular walls. As a result, for example, permanent deformation occurs in a proximal end side (large-diameter portion) of the shaft. This may hinder subsequent operations of the guidewire.

Accordingly, it is an object of the present invention to provide a guidewire that, even when the guidewire is inserted into a blood vessel that is considerably bent, is capable of ensuring high followability and is capable of reducing permanent deformation.

To this end, according to the present invention, there is provided a guidewire as defined solely in independent claim 1 and subsequent dependent claims 2-12.

In one example, the core may comprise a core shaft located at the distal end portion of the core in addition to the second coil body. The core shaft may be any kind of a shaft extending from the distal end of the second coil body. For example, the core shaft may be formed of only a linear portion having a constant diameter or may be formed of only a tapered portion. The second coil body may be joined to a proximal end of the core shaft by a joint covering a boundary between the core shaft and the second coil body. The second coil body may extend from the proximal end of the core.

In the guidewire according to the aspect, at least a part of the core is formed of a second coil body composed of a plurality of wires that are spirally twisted together. In such a second coil body, the wires can move slightly relative to each other. Therefore, in addition to the degree of freedom and the flexibility being high, sufficient restoring force is also ensured.

Therefore, for example, when inserting the guidewire along an inverted U-shaped winding path extending from a blood vessel in the right leg to a blood vessel in the left leg by a cross over method, it becomes possible to easily follow the shapes of blood vessels that are excessively bent. In addition, even if the proximal end portion of the guidewire is excessively bent by a load that is received by the proximal end portion of the guidewire when the proximal end portion of the guidewire, for example, contacts vascular walls, it is less likely for permanent deformation to occur. Consequently, there is no possibility of subsequent operations being hindered. This makes it possible to continuously use the guidewire.

In another example, the core may comprise the second coil body extending to the distal end of the core. According to this guidewire, the wires can move slightly relative to each other over the entire second coil body in a longitudinal direction thereof. Therefore, it is possible to ensure a sufficient degree of freedom, increase flexibility, and ensure sufficient restoring force. The second coil body may extend to the distal end of the core from the proximal end of the core. That is, the second coil body may extend all over the total length of the core. Alternatively, the second coil body may extend from a portion between the proximal end of the core and the distal end of the core to the distal end of the core.

Therefore, for example, when inserting the guidewire along an inverted U-shaped winding path extending from a blood vessel in the right leg to a blood vessel in the left leg by a cross over method, it becomes possible to cause the entire guidewire to easily follow the shapes of blood vessels that are excessively bent. Even if the guidewire is excessively bent by a load that is received by the guidewire when the guidewire, for example, contacts vascular walls, it is possible to reduce permanent deformation over the entire guidewire in the longitudinal direction thereof. Consequently, there is no possibility of subsequent operations being hindered. This makes it possible to continuously use the guidewire.

### BRIEF DESCRIPTION OF THE DRAWINGS

Fig. 1 is a partial sectional enlarged view of a guidewire according to a first embodiment of the present invention.
Fig. 2 is a perspective view of a second coil body of the guidewire according to the first embodiment of the present invention.
Fig. 3 is a partial sectional enlarged view of a guidewire according to a second embodiment of the present invention.
Fig. 4 is a partial sectional enlarged view of a guidewire according to a third embodiment of the present invention.
Fig. 5 is a partial sectional enlarged view of a guidewire according to a fourth embodiment of the present invention.
Fig. 6 is a partial sectional enlarged view of a guidewire according to a fifth embodiment of the present invention.
Fig. 7 is a perspective view of a second coil body of the guidewire according to the fifth embodiment of the present invention.
Fig. 8 is a partial sectional enlarged view of a guidewire according to a sixth embodiment of the present invention.
Fig. 9 is a sectional view taken along line IX-IX of a second coil body in Fig. 8.
Fig. 10 is a partial sectional enlarged view of a guidewire according to a seventh embodiment of the present invention.
Fig. 11 is a sectional view taken along line XI-XI of a second coil body in Fig. 10.
Fig. 12 is a partial sectional enlarged view of a guidewire according to an eighth embodiment of the present invention.
Fig. 13 is a perspective view of a second coil body of the guidewire according to the eighth embodiment of the present invention.
Fig. 14 is a partial sectional enlarged view of a guidewire according to a ninth embodiment of the present invention.
Fig. 15 is a perspective view of a second coil body of the guidewire according to the ninth embodiment of the present invention.
Fig. 16 is a partial sectional enlarged view of a guidewire according to a tenth embodiment of the present invention.
Fig. 17 is a sectional view taken along line XVII-XVII of a second coil body in Fig. 16.
Fig. 18 is a partial sectional enlarged view of a guidewire according to an eleventh embodiment of the present invention.
Fig. 19 is a sectional view taken along line XIX-XIX of a second coil body in Fig. 18.

### DESCRIPTION OF THE PREFERRED EMBODIMENTS

### First Embodiment

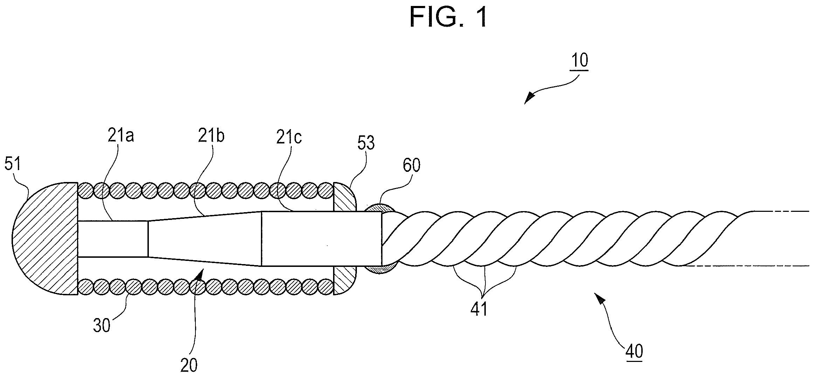

A guidewire 10 according to a first embodiment of the present invention is described with reference to Fig. 1. In Fig. 1, the left side corresponds to a distal end that is inserted into a body and the right side corresponds to a proximal end that is operated by an operator, such as a doctor. Each figure schematically illustrates a guidewire and its dimensional ratios differ from actual dimensional ratios.

The guidewire 10 shown in Fig. 1 is used for, for example, treating blood vessels of the lower limbs by a cross over method. The guidewire 10 includes a core and a first coil body 30. The core is composed of a core shaft 20 and a second coil body 40. The core extends from a proximal end to a distal end. The first coil body 30 is wound around a distal end portion of the core. The second coil body 40 is joined to a proximal end of the core shaft 20. That is, a distal end surface of the second coil body 40 is joined to a proximal end surface of the core shaft 20.

First, the core shaft 20 is described. From the distal end to the proximal end, the core shaft 20 includes a first linear portion 21a, a tapered portion 21b, and a second linear portion 21c. The first linear portion 21a is a portion at the tip of the core shaft 20 and is the most flexible portion of the core shaft 20. The first linear portion 21a is formed into a flat shape by pressing. The tapered portion 21b is circular in cross section. The diameter of the tapered portion 21b decreases towards the distal end. The diameter of the second linear portion 21c is larger than the diameter of the first linear portion 21a. The core shaft 20 is not limited to this structure mentioned above. The core shaft 20 may be any kind of a shaft extending from the distal end of the second coil body 40. For example, the core shaft 20 may be formed of only a linear portion having a constant diameter or may be formed of only a tapered portion.

Materials of the core shaft 20 are not particularly limited to certain materials. Examples of materials include stainless steel (SUS304), a super-elastic alloy (such as a Ni-Ti alloy), a piano wire, and a cobalt-based alloy.

Next, the first coil body 30 is described. The first coil body 30 according to the first embodiment is composed of single-stranded wires spirally wound together. However, the first coil body 30 is not limited to this type. For example, the first coil body 30 may be composed of multi-stranded wires, each being composed of a plurality of strands that are twisted together.

Materials of the first coil body 30 are not particularly limited to certain materials. Examples of materials include stainless steels (such as martensitic stainless steel, ferritic stainless steel, austenitic stainless steel, austenite two-phase stainless steel, ferrite two-phase stainless steel, and precipitation hardening stainless steel), super-elastic alloys (such as a Ni-Ti alloy), platinum, gold, tungsten, tantalum, and iridium (which are metals that are opaque to X rays), and alloys thereof.

As shown in Fig. 1, a distal end of the first coil body 30 is secured to a distal end of the core shaft 20 by a distal-end-side joint 51. A proximal end of the first coil body 30 is secured to the core shaft 20 by a proximal-end-side joint 53. Materials of the distal-end-side joint 51 and the proximal-end-side joint 53 are limited to certain materials which are brazing metal materials such as a Sn-Pb alloy, a Pb-Ag alloy, a Sn-Ag alloy, and an Au-Sn alloy.

In the first embodiment, the proximal end portion of the core shaft 20 (that is, the second linear portion 21c) is exposed from the first coil body 30. The second coil body 40 is joined to the proximal end of the core shaft 20 by a joint 60. The joint 60 is provided outside the first coil body 30. In this embodiment, the joint 60 is formed by brazing using a brazing metal material. The joint 60 is not limited to this and may be formed by welding, or bonding.

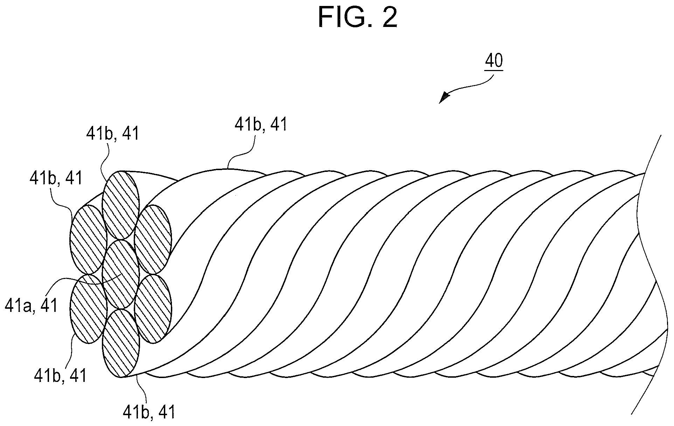

As shown in Fig. 2, the second coil body 40 is composed of a plurality of single-stranded wires 41 that are spirally twisted together. Each of the spirally twisted wires 41 is composed of a single strand 41. More specifically, the second coil body 40 includes a core wire 41a and six side wires 41b that are twisted so as to cover the outer periphery of the core wire 41a.

Materials of the second coil body 40 are not particularly limited to certain materials. Examples of materials include stainless steels (such as martensitic stainless steel, ferritic stainless steel, austenitic stainless steel, austenite two-phase stainless steel, ferrite two-phase stainless steel, and precipitation hardening stainless steel), super-elastic alloys (such as a Ni-Ti alloy), platinum, gold, tungsten, tantalum, and iridium (which are metals that are opaque to X rays), and alloys thereof.

Examples of materials of the joint 60, which joins the second coil body 40 to the proximal end of the core shaft 20, include brazing metal materials such as a Sn-Pb alloy, a Pb-Ag alloy, a Sn-Ag alloy, and an Au-Sn alloy. However, means for joining the second coil body 40 to the proximal end of the core shaft 20 is not particularly limited to certain means. Examples thereof include spot welding using laser and butt resistance welding such as butt seam welding.

In this way, in the first embodiment, the second coil body 40 composed of the plurality of single-stranded wires 41 that are spirally twisted together is joined to the proximal end of the core shaft 20 by the joint 60. In such a second coil body 40, the wires 41 can move slightly relative to each other. Therefore, in addition to the degree of freedom and the flexibility being high, sufficient restoring force is also ensured.

Therefore, for example, when inserting the guidewire 10 along an inverted U-shaped winding path extending from a blood vessel in the right leg to a blood vessel in the left leg by a cross over method, it becomes easy to follow the shapes of blood vessels that are excessively bent. In addition, even if the proximal end portion of the guidewire 10 is excessively bent by a load that is received by the proximal end portion of the guidewire 10 when the proximal end portion of the guidewire 10, for example, contacts vascular walls, it is less likely for permanent deformation to occur. Consequently, there is no possibility of subsequent operations being hindered. This makes it possible to continuously use the guidewire.

### Second Embodiment

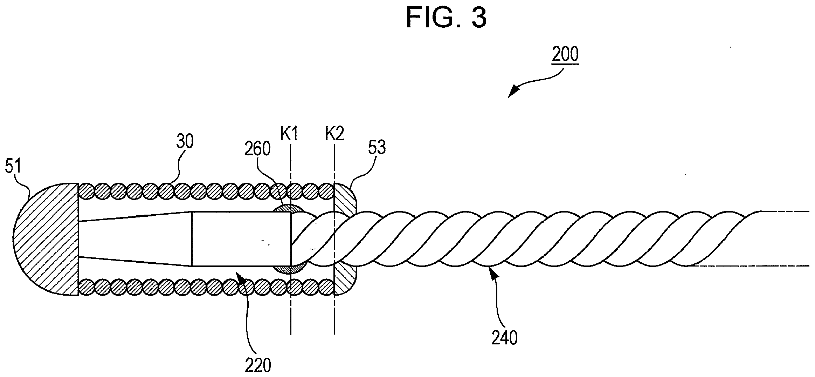

Fig. 3 is a partial sectional enlarged view of a guidewire 200 according to a second embodiment of the present invention. In Fig. 3, the left side corresponds to a distal end that is inserted into a body and the right side corresponds to a proximal end that is operated by an operator, such as a doctor. Structural parts that correspond to those according to the above-described first embodiment are given the same reference numerals and are not described. A description is hereunder given by focusing on the differences.

In the above-described first embodiment, the proximal end of the core shaft 20 is exposed from the first coil body 30 and the portion of the second coil body 40 that is joined to the core shaft 20 (the joint 60) is also exposed at the proximal end side of the first coil body 30. In contrast, in the guidewire 200 according to the second embodiment, a base end K1 of a core shaft 220 is positioned inside the first coil body 30 (that is, closer to a distal end than a proximal end K2 of the first coil body 30) and a portion of a second coil body 240 that is joined to the core shaft 220 (that is, a joint 260) is provided inside the first coil body 30.

According to this guidewire 200, as shown in Fig. 3, even if the joint 260 is provided in a convex manner at a surface of the core shaft 220 and a surface of the second coil body 240, the joint 260 is disposed inside the first coil body 30 while being covered by the first coil body 30. Therefore, there is no possibility of vascular walls being damaged due to contact of the joint 260 with the vascular walls.

Since the portion of the second coil body 240 that is joined to the core shaft 220 (that is, the joint 260) is covered by the first coil body 30, the joint 260 does not contact a hard lesion in a blood vessel, so that it is possible to maintain the joining state between the core shaft 220 and the second coil body 240 in a good state.

### Third Embodiment

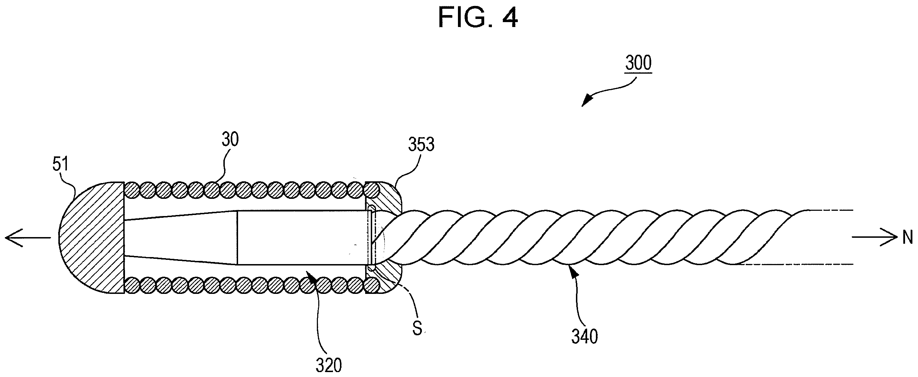

Fig. 4 is a partial sectional enlarged view of a guidewire 300 according to a third embodiment of the present invention. In Fig. 4, the left side corresponds to a distal end that is inserted into a body and the right side corresponds to a proximal end that is operated by an operator, such as a doctor. Structural parts that correspond to those according to the above-described second embodiment are given the same reference numerals and are not described. A description is hereunder given by focusing on the differences.

In the above-described second embodiment, as shown in Fig. 3, the proximal end of the core shaft 220 is positioned in the first coil body 30 (that is, closer to the distal end than the proximal end of the first coil body 30) and a portion of the second coil body 240 that is joined to the core shaft 220 (that is, the joint 260) is provided in the first coil body 30. In contrast, in the guidewire 300 according to the third embodiment, a proximal end of the first coil body 30 and a proximal end of the core shaft 320 are positioned at corresponding locations in a longitudinal direction N and a proximal end joint 353 is provided so as to cover a boundary (interface) S between the core shaft 320 and a second coil body 340. The second coil body 340 is joined to the core shaft 320.

That is, a distal end surface of the second coil body 340 is joined to a proximal end surface of the core shaft 320. The proximal end joint 353 is provided so as to cover the entire periphery of the boundary S. In the third embodiment, this causes the boundary S between the core shaft 320 and the second coil body 340 to be reinforced by the proximal end joint 353 where the proximal end of the first coil body 30 is joined to the core shaft 320.

As a result, for example, when inserting the guidewire 300 along an inverted U-shaped winding path extending from a blood vessel in the right leg to a blood vessel in the left leg by a cross over method, even if the guidewire 300 is largely bent by following such blood vessels, the second coil body 340 does not separate from the core shaft 320, so that it is possible to maintain the joining state of the second coil body 340 with respect to the core shaft 320 in a good state.

### Fourth Embodiment

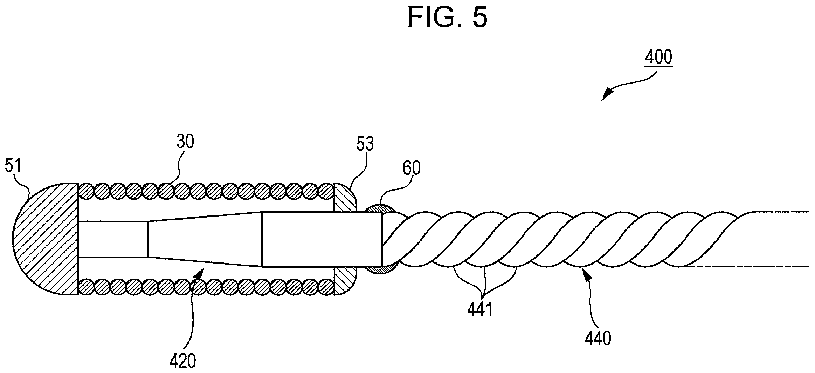

Fig. 5 is a partial sectional enlarged view of a guidewire 400 according to a fourth embodiment of the present invention. In Fig. 5, the left side corresponds to a distal end that is inserted into a body and the right side corresponds to a proximal end that is operated by an operator, such as a doctor. Structural parts that correspond to those according to the above-described first embodiment are given the same reference numerals and are not described. A description is hereunder given by focusing on the differences.

In the guidewire 400 according to the fourth embodiment, the material of wires 441 of a second coil body 440 is the same as the material of a core shaft 420. In the fourth embodiment, from the viewpoint of providing sufficient flexibility and restoring force with respect to bending, it is desirable that the material of the wires 441 of the second coil body 440 and the material of the core shaft 420 both be stainless steel.

Accordingly, by forming the wires 441 of the second coil body 440 out of the same material as the core shaft 420, the second coil body 440 is firmly joined with the core shaft 420, so that, during an operation, the second coil body 440 and the core shaft 420 are not separated from each other, as a result of which safety is ensured.

Although, in the fourth embodiment, an example using the guidewire according to the first embodiment is described, the structure according to the fourth embodiment may be used in each of the second and third embodiments. Even in such cases, the operational advantages according to the fourth embodiment are not affected at all. Similarly, it is possible to maintain the joining state of the second coil body with respect to the core shaft in a good state.

### Fifth Embodiment

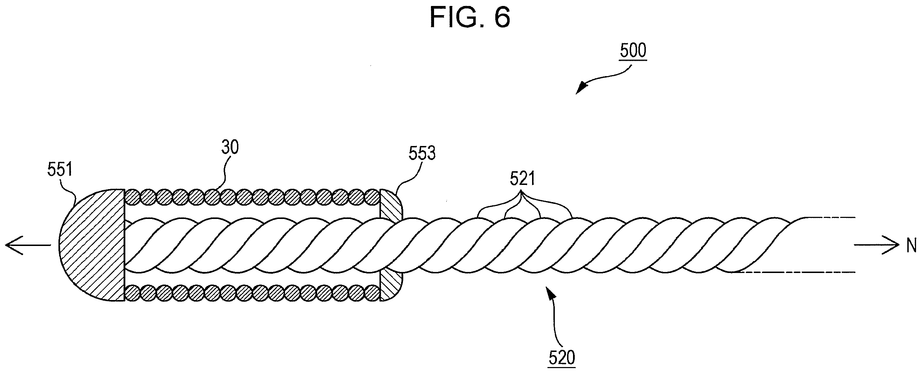

Fig. 6 is a partial sectional enlarged view of a guidewire 500 according to a fifth embodiment of the present invention. In Fig. 6, the left side corresponds to a distal end that is inserted into a body and the right side corresponds to a proximal end that is operated by an operator, such as a doctor. Structural parts that correspond to those according to the above-described first embodiment are given the same reference numerals and are not described. A description is hereunder given by focusing on the differences.

The guidewire 500 according to the fifth embodiment includes a core and a first coil body 30. The core is composed of a second coil body 520. The first coil body 30 is wound around a distal end portion of the second coil body 520. The second coil body 520 extends to the distal end of the core from the proximal end of the core. That is, the second coil body 520 extends all over the total length of the core. Alternatively, the second coil body 520 may extend from a portion between the proximal end of the core and the distal end of the core to the distal end of the core.

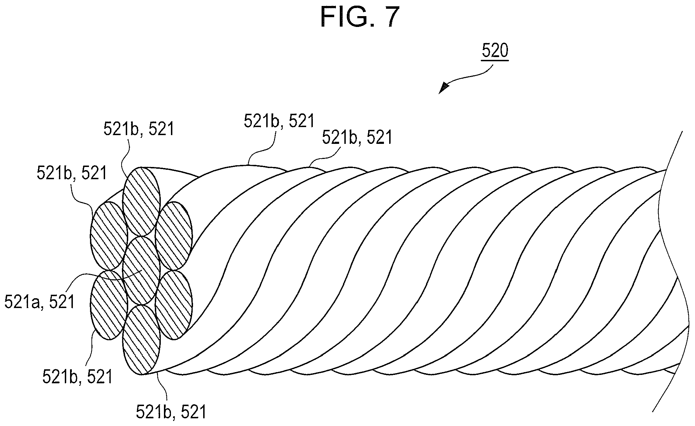

The second coil body 520 is composed of a plurality of single-stranded wires 521 that are spirally twisted together over the entire second coil body 520 in a longitudinal direction N thereof. More specifically, similarly to the second coil body according to the above-described first embodiment, as shown in Fig. 7, the second coil body 520 includes a core wire 521a and six side wires 521b that are twisted so as to cover the outer periphery of the core wire 521a.

A distal end of the coil body 30 is joined to a distal end of the second coil body 520 by a distal-end-side joint 551. A proximal end of the coil body 30 is joined to a substantially intermediate portion of the second coil body 520 by a proximal-end-side joint 553.

Accordingly, according to the guidewire 500 including the second coil body 520 composed of the plurality of single-stranded wires 521 that are spirally twisted together over the entire guidewire 500 in the longitudinal direction N, the single-stranded wires 521 can move slightly relative to each other over the entire second coil body 520 in the longitudinal direction thereof. Therefore, it is possible to ensure a sufficient degree of freedom, increase flexibility, and ensure sufficient restoring force.

Consequently, for example, when inserting the guidewire 500 along an inverted U-shaped winding path extending from a blood vessel in the right leg to a blood vessel in the left leg by a cross over method, it becomes possible to cause the entire guidewire 500 to easily follow the shapes of blood vessels that are excessively bent. Even if the guidewire 500 is excessively bent by a load that is received by the guidewire 500 when the guidewire 500, for example, contacts vascular walls, it is possible to reduce permanent deformation over the entire guidewire 500 in the longitudinal direction N thereof. Consequently, there is no possibility of subsequent operations being hindered. This makes it possible to continuously use the guidewire 500.

### Sixth Embodiment

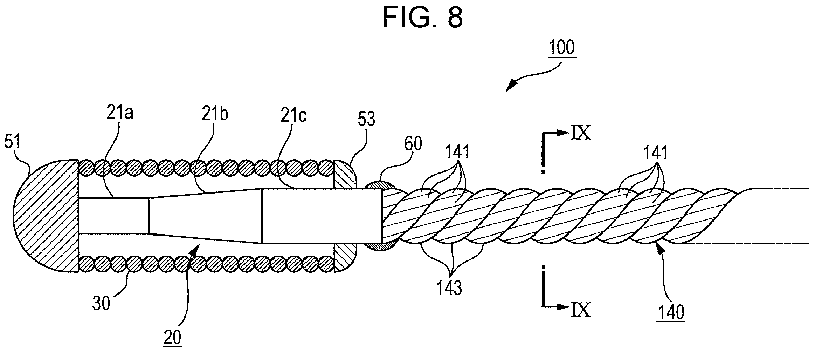

Fig. 8 is a partial sectional enlarged view of a guidewire 100 according to a sixth embodiment of the present invention. In Fig. 8, the left side corresponds to a distal end that is inserted into a body and the right side corresponds to a proximal end that is operated by an operator, such as a doctor. Structural parts that correspond to those according to the above-described first embodiment are given the same reference numerals and are not described. A description is hereunder given by focusing on the differences.

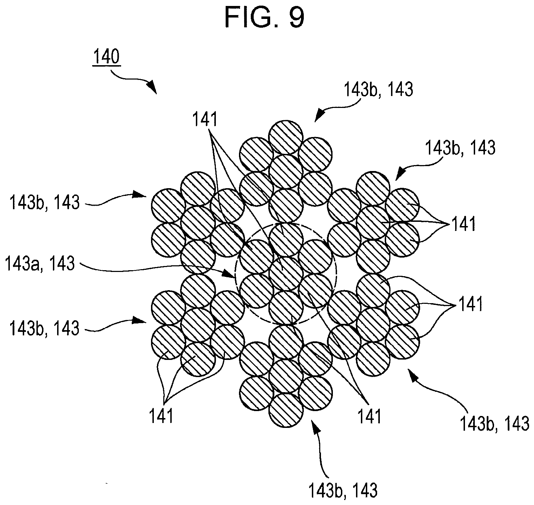

Fig. 9 is a sectional view taken along line IX-IX of a second coil body 140 in Fig. 8. Fig. 9 schematically illustrates the second coil body 140. The cross-sectional shape of the second coil body 140 has dimensional ratios that differ from actual dimensional ratios.

In the above-described first embodiment, each of the plurality of wires (six wires in the first embodiment) that are spirally twisted together is composed of a sigle strand. In contrast, the guidewire 100 according to the sixth embodiment uses the second coil body 140 in which a plurality of multi-stranded wires 143, each being composed of a plurality of strands 141 that are twisted together, are spirally twisted together.

More specifically, as shown in Fig. 9, the second coil body 140 includes a multi-stranded core wire 143a, which is a core member, and six multi-stranded side wires 143b, which are spirally twisted so as to cover the outer periphery of the multi-stranded core wire 143a. The multi-stranded core wire 143a and the multi-stranded side wires 143b all have the same structure.

Materials of the second coil body 140 are not particularly limited to certain materials. Examples of materials include stainless steels (such as martensitic stainless steel, ferritic stainless steel, austenitic stainless steel, austenite two-phase stainless steel, ferrite two-phase stainless steel, and precipitation hardening stainless steel), super-elastic alloys (such as a Ni-Ti alloy), platinum, gold, tungsten, tantalum, and iridium (which are metals that are opaque to X rays), and alloys thereof.

Accordingly, in the guidewire 100 in which the second coil body 140 (which includes the multi-stranded wires 143, each being composed of the plurality of strands 141 twisted together, that are spirally twisted together) is joined to a proximal end of the core shaft 20, in addition to making it possible for adjacent multi-stranded wires 143 to move slightly relative to each other, it is also possible for the strands 141 of the multi-stranded wires 143 to move slightly relative to each other. Therefore, compared to the above-described first embodiment, the degree of freedom is further increased and sufficient flexibility is ensured.

Consequently, for example, when inserting the guidewire 100 along an inverted U-shaped winding path extending from a blood vessel in the right leg to a blood vessel in the left leg by a cross over method, the guidewire 100 can very easily follow such blood vessels that are excessively bent, so that operability is increased.

Further, at the proximal end of the guidewire 100 (the second coil body 140), at the same time that the strands 141 are pressed together due to the application of rotational force, the multi-stranded wires 143 are also pressed together, as a result of which contact pressure and thus contact therebetween is increased. As a result, sufficient torque is reliably transmitted towards the distal end and the guidewire 100 is pushed well into blood vessels in the legs that are excessively bent.

Although, in the sixth embodiment, an example using the guidewire according to the first embodiment is described, the structure according to the sixth embodiment may be used in each of the second to fourth embodiments. Even in such cases, the operational advantages according to the sixth embodiment are not affected at all. Similarly, it is possible to ensure high operability of the guidewire.

### Seventh Embodiment

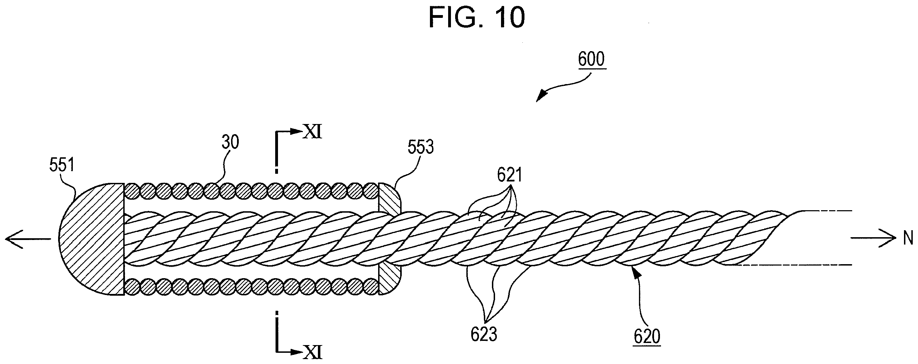

Fig. 10 is a partial sectional enlarged view of a guidewire 600 according to a seventh embodiment of the present invention. In Fig. 10, the left side corresponds to a distal end that is inserted into a body and the right side corresponds to a proximal end that is operated by an operator, such as a doctor. Structural parts that correspond to those according to the above-described fifth embodiment are given the same reference numerals and are not described. A description is hereunder given by focusing on the differences.

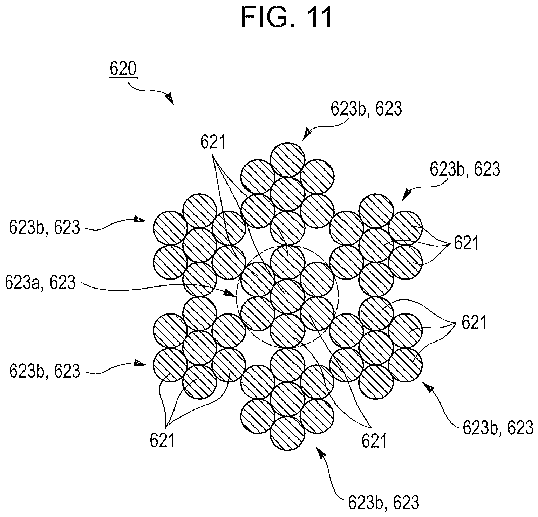

Fig. 11 is a sectional view taken along line XI-XI of a second coil body 620 in Fig. 10. Fig. 11 schematically illustrates the second coil body 620. The cross-sectional shape of the second coil body 620 has dimensional ratios that differ from actual dimensional ratios.

In the above-described fifth embodiment, the second coil body 520 is formed of a plurality of single-stranded wires 521 that are spirally twisted together (refer to Fig. 6). In contrast, the guidewire 600 according to the seventh embodiment uses the second coil body 620 in which a plurality of multi-stranded wires 623, each being composed of a plurality of strands 621 that are twisted together, are spirally twisted together.

More specifically, as shown in Fig. 11, the second coil body 620 includes a multi-stranded core wire 623a, which is a core member, and six multi-stranded side wires 623b, which are spirally twisted so as to cover the outer periphery of the multi-stranded core wire 623a. The multi-stranded core wire 623a and the multi-stranded side wires 623b all have the same structure.

Accordingly, according to the guidewire 600 including the second coil body 620 including the plurality of multi-stranded wires 623 (each being composed of the plurality of strands 621 that are twisted together) that are spirally twisted together over the entire guidewire 600 in a longitudinal direction N, in addition to making it possible for adjacent multi-stranded wires 623 to move slightly relative to each other, it is also possible for the strands 621 of the multi-stranded wires 623 to move slightly relative to each other. Therefore, compared to the above-described fifth embodiment, the degree of freedom is further increased and sufficient flexibility is ensured.

Therefore, for example, when inserting the guidewire 600 along an inverted U-shaped winding path extending from a blood vessel in the right leg to a blood vessel in the left leg by a cross over method, it becomes possible to cause the entire guidewire 600 to more easily follow the shapes of blood vessels that are excessively bent. Even if the guidewire 600 is excessively bent by a load that is received by the guidewire 600 when the guidewire 600, for example, contacts vascular walls, it is possible to reliably reduce permanent deformation over the entire guidewire 600 in the longitudinal direction N thereof. Consequently, there is no possibility of subsequent operations being hindered. This makes it possible to continuously use the guidewire 600.

### Eighth Embodiment

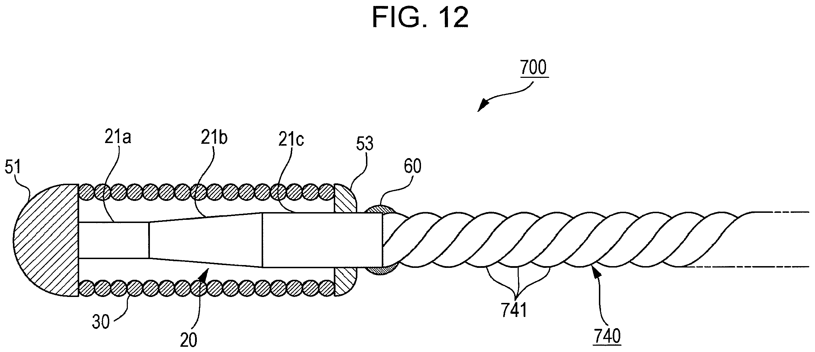

Fig. 12 is a partial sectional enlarged view of a guidewire 700 according to an eighth embodiment of the present invention. In Fig. 12, the left side corresponds to a distal end that is inserted into a body and the right side corresponds to a proximal end that is operated by an operator, such as a doctor. Structural parts that correspond to those according to the above-described first embodiment are given the same reference numerals and are not described. A description is hereunder given by focusing on the differences.

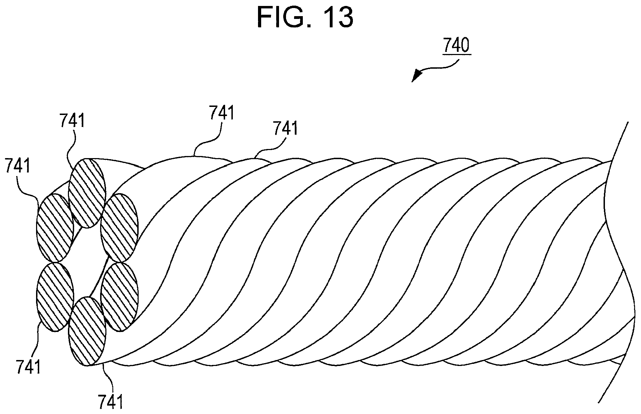

In the first embodiment, the second coil body 40 including the core wire 41a and six side wires 41b that are twisted so as to cover the outer periphery of the core wire 41a is used (refer to Fig. 2). In contrast, in the guidewire 700 according to the eighth embodiment, as shown in Fig. 13, a second coil body 740 that has a hollow and that does not include a core wire is used. That is, the second coil body 740 according to the eighth embodiment is composed of six single-stranded wires 741 that are spirally twisted together around a hollow.

According to the eighth embodiment, since a gap is provided in the center of the second coil body 740, compared to the first embodiment, the flexibility of the second coil body 740 is further increased. As a result, for example, when inserting the guidewire 700 along an inverted U-shaped winding path extending from a blood vessel in the right leg to a blood vessel in the left leg by a cross over method, it becomes possible to cause the guidewire 700 to reliably follow the blood vessels that are excessively bent. Consequently, the operability of the guidewire 700 is further increased.

Although, in the eighth embodiment, an example using the guidewire according to the first embodiment is described, the structure according to the eighth embodiment may be used in each of the second to fourth embodiments. Even in such cases, the operational advantages according to the eighth embodiment are not affected at all. Similarly, it is possible to ensure high operability of the guidewire.

### Ninth Embodiment

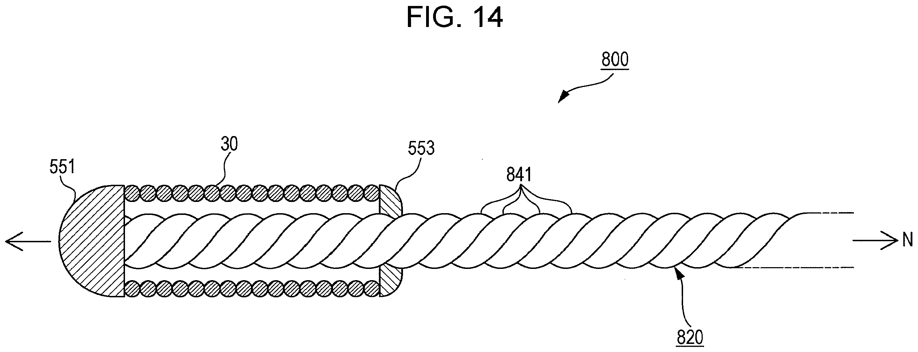

Fig. 14 is a partial sectional enlarged view of a guidewire 800 according to a ninth embodiment of the present invention. In Fig. 14, the left side corresponds to a distal end that is inserted into a body and the right side corresponds to a proximal end that is operated by an operator, such as a doctor. Structural parts that correspond to those according to the above-described fifth embodiment are given the same reference numerals and are not described. A description is hereunder given by focusing on the differences.

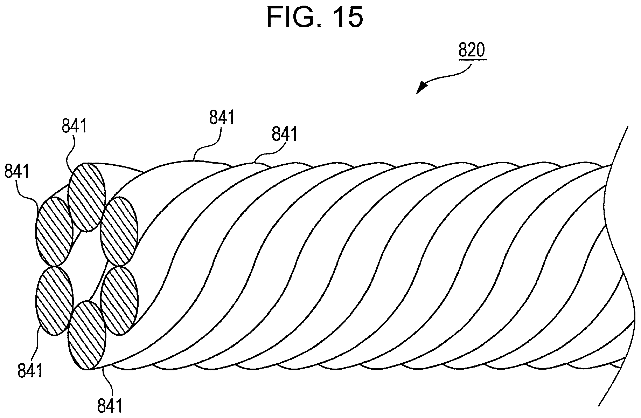

In the above-described fifth embodiment, the second coil body 520 including the core wire 521a and six side wires 521b that are twisted so as to cover the outer periphery of the core wire 521a is used (refer to Fig. 7). In contrast, in the guidewire 800 according to the ninth embodiment, as shown in Fig. 15, a second coil body 820 that has a hollow and that does not include a core wire is used. That is, the second coil body 820 according to the ninth embodiment is composed of six single-stranded wires 841 that are spirally twisted together around a hollow.

According to the ninth embodiment, since a gap is provided in the center of the second coil body 820, compared to the fifth embodiment, the flexibility of the second coil body 820 is further increased. That is, the guidewire 800 according to the ninth embodiment is provided with sufficient flexibility over the entire guidewire 800 in a longitudinal direction N thereof. As a result, for example, when inserting the guidewire 800 along an inverted U-shaped winding path extending from a blood vessel in the right leg to a blood vessel in the left leg by a cross over method, it becomes possible to cause the entire guidewire 800 to reliably follow the blood vessels that are excessively bent. Consequently, the operability of the guidewire 800 is further increased.

### Tenth Embodiment

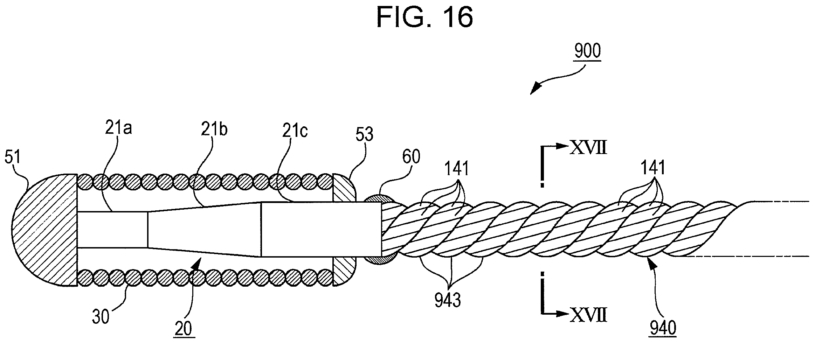

Fig. 16 is a partial sectional enlarged view of a guidewire 900 according to a tenth embodiment of the present invention. In Fig. 16, the left side corresponds to a distal end that is inserted into a body and the right side corresponds to a proximal end that is operated by an operator, such as a doctor. Structural parts that correspond to those according to the above-described sixth embodiment are given the same reference numerals and are not described. A description is hereunder given by focusing on the differences.

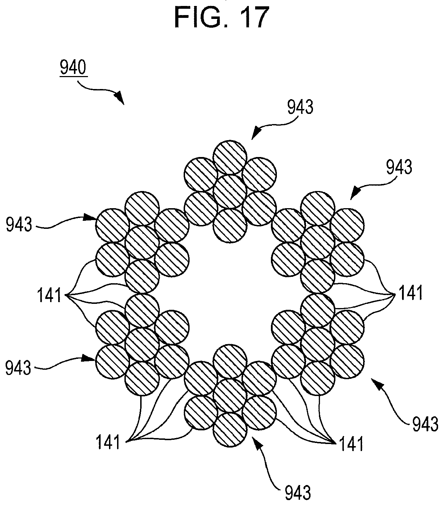

Fig. 17 is a sectional view taken along line XVII-XVII of a second coil body 940 in Fig. 16. Fig. 17 schematically illustrates the second coil body 940. The cross-sectional shape of the second coil body 940 has dimensional ratios that differ from actual dimensional ratios.

In the above-described sixth embodiment, the second coil body 140 including the multi-stranded core wire 143a, which is a core wire, and six multi-stranded side wires 143b, which are spirally twisted so as to cover the outer periphery of the multi-stranded core wire 143a, is used (refer to Fig. 9). In contrast, in the guidewire 900 according to the tenth embodiment, as shown in Fig. 17, the second coil body 940 that has a hollow and that does not include a core member at the center is used. That is, in the tenth embodiment, the second coil body 940 having a hollow and including six multi-stranded wires 943 that are spirally twisted together is used.

According to the tenth embodiment, since a gap is provided in the center of the second coil body 940, compared to the sixth embodiment, the flexibility of the second coil body 940 is further increased. As a result, for example, when inserting the guidewire 900 along an inverted U-shaped winding path extending from a blood vessel in the right leg to a blood vessel in the left leg by a cross over method, it becomes possible to cause the guidewire 900 to more reliably follow the blood vessels that are excessively bent. Consequently, the operability of the guidewire 900 is further increased.

### Eleventh Embodiment

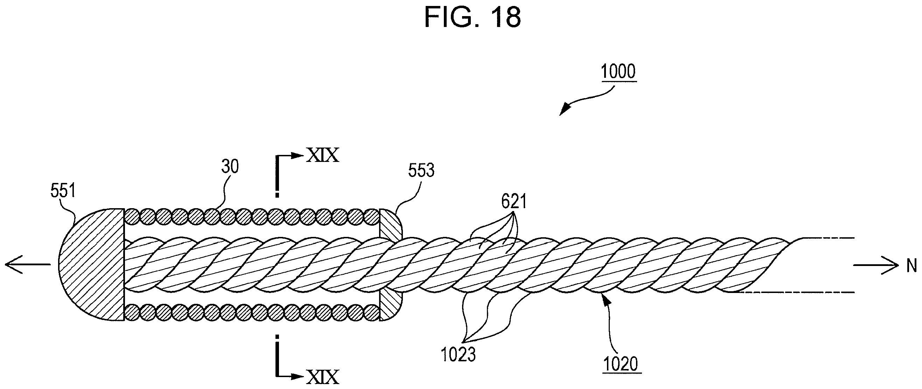

Fig. 18 is a partial sectional enlarged view of a guidewire 1000 according to an eleventh embodiment of the present invention. In Fig. 18, the left side corresponds to a distal end that is inserted into a body and the right side corresponds to a proximal end that is operated by an operator, such as a doctor. Structural parts that correspond to those according to the above-described seventh embodiment are given the same reference numerals and are not described. A description is hereunder given by focusing on the differences.

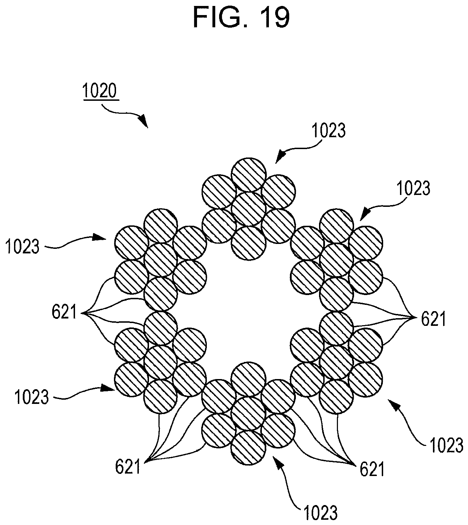

Fig. 19 is a sectional view taken along line XIX-XIX of a second coil body 1020 in Fig. 18. Fig. 19 schematically illustrates the second coil body 1020. The cross-sectional shape of the second coil body 1020 has dimensional ratios that differ from actual dimensional ratios.

In the above-described seventh embodiment, the second coil body 620 including the multi-stranded core wire 623a, which is a core wire, and six multi-stranded side wires 623b, which are spirally twisted so as to cover the outer periphery of the multi-stranded core wire 623a, is used (refer to Fig. 11). In contrast, in the guidewire 1000 according to the eleventh embodiment, as shown in Fig. 19, the second coil body 1020 that has a hollow and that does not include a core member is used. That is, in the eleventh embodiment, the second coil body 1020 having a hollow and including six multi-stranded wires 1023 that are spirally twisted together is used.

According to the eleventh embodiment, since a gap is provided in the center of the second coil body 1020, compared to the above-described seventh embodiment, the flexibility of the second coil body 1020 is further increased. That is, the guidewire 1000 according to the eleventh embodiment is provided with sufficient flexibility over the entire guidewire 1000 in a longitudinal direction N thereof. As a result, for example, when inserting the guidewire 1000 along an inverted U-shaped winding path extending from a blood vessel in the right leg to a blood vessel in the left leg by a cross over method, it becomes possible to cause the entire guidewire 1000 to reliably flexibly follow the blood vessels that are excessively bent. Consequently, the operability of the guidewire 1000 is further increased.

## Claims

1. A guidewire (10,100,200,300,400,500,600,700,800,900,1000) comprising:
a core extending from a proximal end to a distal end;
a first coil body (30) that is wound around a distal end portion of the core, and at least a part of the core is formed of
a second coil body (40,140,240,340,440,520,620,740,820,940,1020) composed of a plurality of wires (41,143,441,521,623,741,841,943,1023) that are spirally twisted together, **characterised in that**
a distal end of the first coil body (30) is secured to a distal end of the core by a distal-end-side joint (51,551)
a proximal end of the first coil body (30) is secured to the core by a proximal-end-side joint (53,353,553); and
metal materials of the distal-end-side joint (51,551) and the proximal-end-side joint (53,353,553) are brazing metal materials.

2. The guidewire (10,100,200,300,400,700,900) according to Claim 1, wherein the core comprises a core shaft (20,220,320,420) located at the distal end portion of the core in addition to the second coil body (40,240,340,440,740,940), the second coil body (40,240,340,440,740,940) being joined to a proximal end of the core shaft (20,220,320,420) by a joint (60,260,353) covering a boundary (S) between the core shaft (20,220,320,420) and the second coil body (40,240,340,440,740,940).

3. The guidewire (200,300) according to Claim 2, wherein the joint (260) is provided inside the first coil body (30).

4. The guidewire (300) according to Claim 2 or 3, wherein a proximal end of the first coil body (30) is secured to the core by a proximal end side joint (353) covering the boundary (S).

5. The guidewire (10,100,400,700,900) according to Claim 2, wherein the joint (60) is provided outside the first coil body (30).

6. The guidewire (10,100,200,300,400,700,900) according to any one of Claims 2 to 5, wherein a material of the second coil body (40,140,240,340,440,740,940) is the same as a material of the core shaft (20,220,320,420).

7. The guidewire (10,100,200,300,400,700,900) according to any one of Claims 2 to 6, wherein the joint (60,260,353) is formed by brazing, welding, or bonding.

8. The guidewire (500,600,800,1000) according to Claim 1, wherein the core comprises the second coil body (520,620,820,1020) extending to the distal end of the core.

9. The guidewire (10,200,300,400,500,700,800) according to any one of Claims 1 to 8, wherein each of the spirally twisted wires (41,441,521,741,841) is composed of a single strand (41,441,521,741,841).

10. The guidewire (100,600,900,1000) according to any one of Claims 1 to 8, wherein each of the spirally twisted wires (143,623,943,1023) is composed of a plurality of strands (141,621) that are twisted together.

11. The guidewire (10,100,200,300,400,500,600) according to any one of claims 1 to 10, wherein the second coil body (40,140,240,340,440,520,620) includes a core wire (41a,143a,521a,623a) and side wires (41b,143b,521b,623b) covering the outer periphery of the core wire (41a,143a,521a,623a).

12. The guidewire (700,800,900,1000) according to any one of claims 1 to 10, wherein the second coil body (740,820,940,1020) is a hollow body.

## Patentansprüche

1. Führungsdraht (10,100,200,300,400,500,600,700,800,900,1000) mit:
einem Kern, der sich von einem proximalen Ende bis zu einem distalen Ende erstreckt; und
einem ersten Wicklungskörper (30), der um den distalen Endabschnitt des Kerns gewickelt ist, wobei
wenigstens ein Teil des Kerns aus einem zweiten Wicklungskörper (40,140,240,340,440,520,620,740,820,940,1020) gebildet ist, der aus einer Vielzahl von miteinander schraubenförmig verdrillten Drähten (41,143,441,521,623,741,841,943,1023) besteht, **dadurch gekennzeichnet, dass**
ein distales Ende des ersten Wicklungskörpers (30) durch eine distale Fügestelle 51,551) an einem distalen Ende des Kerns befestigt ist,
ein proximales Ende des ersten Wicklungskörpers (30) durch eine proximale Fügestelle (53,353,553) am Kern befestigt ist; und
metallische Werkstoffe der distalen Fügestelle (51,551) und proximalen Fügestelle (53,353,553) metallische Lötwerkstoffe sind.

2. Führungsdraht (10,100,200,300,400,700,900) nach Anspruch 1, wobei der Kern neben dem zweiten Wicklungskörper (40,240,340,440,740,940) einen am distalen Endabschnitt des Kerns angeordneten Kernschaft (20,220,320,420) aufweist, und wobei der zweite Wicklungskörper (40,240,340,440,740,940) durch eine Fügestelle (60,260,353), die die Schnittstelle (S) zwischen dem Kernschaft (20,220,320,420) und dem zweiten Wicklungskörper (40,240,340,440,740,940) ummantelt, an dem proximalen Ende des Kernschafts (20,220,320,420) angefügt ist.

3. Führungsdraht (200,300) nach Anspruch 2, wobei die Fügestelle (260) innerhalb des ersten Wicklungskörpers (30) vorgesehen ist.

4. Führungsdraht (300) nach Anspruch 2 oder 3, wobei das proximale Ende des ersten Wicklungskörpers (30) durch eine proximale Fügestelle (353), die die Schnittstelle (S) ummantelt, am Kern befestigt ist.

5. Führungsdraht (10,100,400,700,900) nach Anspruch 2, wobei die Fügestelle (60) außerhalb des ersten Wicklungskörpers (30) vorgesehen ist.

6. Führungsdraht (10,100,200,300,400,700,900) nach einem der Ansprüche 2 bis 5, wobei der Werkstoff des zweiten Wicklungskörpers (40,140,240,340,440,740,940) der gleiche ist wie der Werkstoff des Kernschafts (20,220,320,420).

7. Führungsdraht (10,100,200,300,400,700,900) nach einem der Ansprüche 2 bis 6, wobei die Fügestelle (60,260,353) durch Verlöten, Verschweißen oder Verkleben ausgebildet ist.

8. Führungsdraht (500,600,800,1000) nach Anspruch 1, wobei der Kern den zweiten Wicklungskörper (520,620,820,1020) aufweist, der sich bis zum distalen Ende des Kerns erstreckt.

9. Führungsdraht (10,200,300,400,500,700,800) nach einem der Ansprüche 1 bis 8, wobei jeder der schraubenförmig verdrillten Drähte (41,441,521,741,841) aus einem einzelnen Strang (41,441,521,741,841) besteht.

10. Führungsdraht (100,600,900,1000) nach einem der Ansprüche 1 to 8, wobei jeder der schraubenförmig verdrillten Drähte (143,623,943,1023) aus einer Vielzahl von miteinander verdrillten Strängen (141,621) besteht.

11. Führungsdraht (10,100,200,300,400,500,600) nach einem der Ansprüche 1 bis 10, wobei der zweite Wicklungskörper (40,140,240,340,440,520,620) einen Kerndraht (41a,143a,521a,623a) und Seitendrähte (41b,143b,521b,623b) aufweist, die den Außenumfang des Kerndrahts (41a,143a,521a,623a) ummanteln.

12. Führungsdraht (700,800,900,1000) nach einem der Ansprüche 1 bis 10, wobei der zweite Wicklungskörper (740,820,940,1020) ein Hohlkörper ist.

## Revendications

1. Fil-guide (10, 100, 200, 300, 400, 500, 600, 700, 800, 900, 1000) comprenant :
une âme s'étendant d'une extrémité proximale à une extrémité distale ;
un premier corps de bobine (30) qui est enroulé autour d'une partie d'extrémité distale de l'âme, et au moins une partie de l'âme est formée avec un second corps de bobine (40, 140, 240, 340, 440, 520, 620, 740, 820, 940, 1020) composé d'une pluralité de fils (41, 143, 441, 521, 623, 741, 841, 943, 1023) qui sont torsadés ensemble en spirale, **caractérisé en ce que** :
une extrémité distale du premier corps de bobine (30) est fixée à une extrémité distale de l'âme par un joint du côté de l'extrémité distale (51, 551),
une extrémité proximale du premier corps de bobine (30) est fixée à l'âme par un joint du côté de l'extrémité proximale (53, 353, 553) ; et
des matériaux métalliques du joint du côté de l'extrémité distale (51, 551) et du joint du côté de l'extrémité proximale (53, 353, 553) sont des matériaux métalliques de brasage.

2. Fil-guide (10, 100, 200, 300, 400, 700, 900) selon la revendication 1, dans lequel l'âme comprend une tige d'âme (20, 220, 320, 420) positionnée au niveau de la partie d'extrémité distale de l'âme en plus du second corps de bobine (40, 240, 340, 440, 740, 940), le second corps de bobine (40, 240, 340, 440, 740, 940) étant assemblé à une extrémité proximale de la tige d'âme (20, 220, 320, 420) par un joint (60, 260, 353) recouvrant une limite (S) entre la tige d'âme (20, 220, 320, 420) et le second corps de bobine (40, 240, 340, 440, 740, 940).

3. Fil-guide (200, 300) selon la revendication 2, dans lequel le joint (260) est prévu à l'intérieur du premier corps de bobine (30).

4. Fil-guide (300) selon la revendication 2 ou 3, dans lequel une extrémité proximale du premier corps de bobine (30) est fixée sur l'âme par un joint du côté de l'extrémité proximale (353) recouvrant la limite (S).

5. Fil-guide (10, 100, 400, 700, 900) selon la revendication 2, dans lequel le joint (60) est prévu à l'extérieur du premier corps de bobine (30).

6. Fil-guide (10, 100, 200, 300, 400, 700, 900) selon l'une quelconque des revendications 2 à 5, dans lequel un matériau du second corps de bobine (40, 140, 240, 340, 440, 740, 940) est le même qu'un matériau de la tige d'âme (20, 220, 320, 420).

7. Fil-guide (10, 100, 200, 300, 400, 700, 900) selon l'une quelconque des revendications 2 à 6, dans lequel le joint (60, 260, 353) est formé par brasage, soudage ou collage.

8. Fil-guide (500, 600, 800, 1000) selon la revendication 1, dans lequel l'âme comprend un second corps de bobine (520, 620, 820, 1020) s'étendant vers l'extrémité distale de l'âme.

9. Fil-guide (10, 200, 300, 400, 500, 700, 800) selon l'une quelconque des revendications 1 à 8, dans lequel chacun des fils torsadés en spirale (41, 441, 521, 741, 841) est composé d'un seul brin (41, 441, 521, 741, 841).

10. Fil-guide (100, 600, 900, 1000) selon l'une quelconque des revendications 1 à 8, dans lequel chacun des fils torsadés en spirale (143, 623, 943, 1023) est composé d'une pluralité de brins (141, 621) qui sont torsadés ensemble.

11. Fil-guide (10, 100, 200, 300, 400, 500, 600) selon l'une quelconque des revendications 1 à 10, dans lequel le second corps de bobine (40, 140, 240, 340, 440, 520, 620) comprend un fil d'âme (41a, 143a, 521a, 623a) et des fils latéraux (41b, 143b, 521b, 623b) recouvrant la périphérie externe du fil d'âme (41a, 143a, 521a, 623a).

12. Fil-guide (700, 800, 900, 1000) selon l'une quelconque des revendications 1 à 10, dans lequel le second corps de bobine (740, 820, 940, 1020) est un corps creux.
